Europäisches Patentamt

European Patent Office

Office européen des brevets

(11) Veröffentlichungsnummer: **0 421 175 A1**

(12) **EUROPÄISCHE PATENTANMELDUNG**

(21) Anmeldenummer: 90117794.9

(22) Anmeldetag: **15.09.90**

(51) Int. Cl.⁵: **G01N 21/03**, G01N 33/48, A61B 5/14

(30) Priorität: **02.10.89 CH 3572/89**

(43) Veröffentlichungstag der Anmeldung: **10.04.91 Patentblatt 91/15**

(84) Benannte Vertragsstaaten: **BE CH DE ES FR GB IT LI NL**

(71) Anmelder: **F. HOFFMANN-LA ROCHE AG Postfach 3255 CH-4002 Basel(CH)**

(72) Erfinder: **Bolliger, Jean-Pierre**

Brühlgasse 7
CH-4153 Reinach(CH)
Erfinder: **Rebstein, Jean-Jacques**
Bündtenweg 17
CH-4104 Oberwil(CH)
Erfinder: **Zaugg, Hans**
Obere Flühackerstrasse 7
CH-4402 Frenkendorf(CH)

(74) Vertreter: **Ventocilla, Abraham et al**
Grenzacherstrasse 124 Postfach 3255
CH-4002 Basel(CH)

(54) **Kapillarröhrchen zur Entnahme von Blutproben, Verfahren zu dessen Herstellung sowie eine Vorrichtung zur Messung der Gerinnungszeit.**

㉟ Kapillarröhrchen zur Entnahme von kapillären Blutproben für ein System zur Messung der Gerinnungszeit.

Zur Verringerung der Infektionsgefahr für das Laborpersonal und zur Vereinfachung des Arbeitsaufwandes für die Entnahme und Aufbereitung der kapillären Blutproben ist das Kapillarröhrchen dadurch gekennzeichnet, dass es aus einem Kunststoff besteht, und in seinem Innenraum und über seine ganze Länge ein Lyophilisat (14, 15, 16) annähernd gleichmässig verteilt ist, das durch Gefriertrocknung eines Gemisches erzeugt wird, das ein Antikoagulans und wenigstens einen hochmolekularen Zusatz enthält.

Fig. 1

Fig. 2

Fig. 3

EP 0 421 175 A1

## KAPILLARRÖHRCHEN ZUR ENTNAHME VON BLUTPROBEN, VERFAHREN ZU DESSEN HERSTELLUNG SOWIE EINE VORRICHTUNG ZUR MESSUNG DER GERINNUNGSZEIT

Die Erfindung betrifft insbesondere ein Kapillarröhrchen zur Entnahme von kapillären Blutproben sowie ein Verfahren zur dessen Herstellung.

Die Erfindung betrifft ausserdem eine Vorrichtung zur Messung der Gerinnungszeit einer kapillären Blutprobe, welche Vorrichtung ein erfindungsgemässes Kapillarröhrchen und eine Küvette zur photometrischen Messung der Gerinnungszeit einer Blutprobe enthält, welche Küvette aus einem Kunststoff besteht und deren oberer Teil durch eine Trennwand in zwei Räumen aufgeteilt ist, welche Trennwand eine vom Boden der Küvette beabstandete scharfe Kante aufweist. Eine solche Küvette sowie eine automatisch durchgeführte Messung der Gerinnungszeit sind in der amerikanischen Patentschrift Nr. 4,659,550 beschrieben.

Das bisher verwendete Verfahren zur Entnahme und Vorbereitung einer kapillären Blutprobe vor der Messung der Gerinnungszeit nach dem konventionellen Mikroquick-Verfahren umfasst mehrere Schritte und erfordert einen relativ grossen Arbeits- und Zeitaufwand im Vergleich zu der im übrigen automatisch durchgeführten Messung. Das bisher verwendete Verfahren zur Entnahme und Vorbereitung der Probe umfasst z.B. folgende Schritte:

eine gerinnungshemmende Lösung , z.B. eine Citratlösung, wird bis zur 0.005 ml-Marke in eine Kapillare aufgezogen,

- durch Einstich mit einer Lanzette wird von einem Finger ein Blutstropfen entnommen, indem die ausführende Person durch Mundpipettierung Blut in die mit der Citratlösung versehene Kapillare bis zur 0.050 ml-Marke einfliessen lässt,

- der Inhalt der Kapillare wird dann sofort von der ausführenden Person in ein Glas- oder Plastikröhrchen gegen dessen Boden ausgeblasen, und

- anschliessend wird durch eine kreisende Bewegung des Röhrchens eine bessere Mischung der Blutprobe mit der Citratlösung erzielt.

Es ist bei den bisherigen Messverfahren üblich, das so vorbereitete Gemisch einer Blutprobe mit einer Citratlösung durch eine Mundpipettierung in die Messküvette einzugeben. Eine Mundpipettierung birgt aber eine nicht unbeachtliche Infektionsgefahr für die ausführende Person.

Es sei in diesem Zusammenhang darauf hingewiesen, dass aus der amerikanischen Patentschrift Nr. 4,756,884 eine Testvorrichtung bekannt ist, bei der u.a.:

- eine flüssige Probe mittels der von einem Kapillarröhrchen ausgeübten Kapillarkraft gesaugt wird,

- eine solche Kapillarkraft zum Mischen der gesaugten Probe mit einem Reagenz eingesetzt wird, das im Kapillarröhrchen enthalten ist, und

- Ein Reagenz in Form eines auflösbaren Gels oder Schwamms zu verwenden, das in der Testvorrichtung enthalten ist.

Gemäss der amerikanischen Patentschrift Nr. 4,756,884 ist das in der Testvorrichtung verwendete Reagenz nur in einem sehr kleinen Bereich der Vorrichtung vorhanden. Deshalb ist eine gute, d.h. eine gleichmässige Mischung der ganzen, in der Testvorrichtung aufgenommene Blutprobe mit dem Reagenz mittels der Kapillarkraft nicht erzielbar. Die in der amerikanischen Patentschrift Nr.4,756,884 enthaltene Lehre ist daher für die Vorbereitung einer Blutprobe vor einer Messung der Blutgerinnungszeit ungeeignet, bei der die Blutprobe zunächst mit einem Antikoagulans gemischt werden soll. Bei einer solchen Vorbereitung ist es nämlich sehr wichtig, eine gleichmässige Mischung der ganzen Blutprobe mit dem Antikoagulans zu erzielen. Wird dies nicht erreicht, kann bereits ein Teil der Blutprobe koagulieren, d.h. ein Gerinnsel bilden, bevor sie in die Messküvette eingegeben wird. In diesem Fall ist die Genauigkeit und sogar die Zuverlässigkeit der Messung ernsthaft in Frage gestellt

Der Erfindung liegt daher in erster Linie die Aufgabe zugrunde, eine Vorrichtung zu schaffen, mit der die obigen Nachteile der bisher bekannten Vorrichtungen beseitigt werden können, wobei insbesondere eine erhebliche Verringerung der Infektionsgefahr für das Laborpersonal und eine starke Vereinfachung des Arbeitsaufwandes für die Entnahme und Aufbereitung der Blutproben sowie für die Durchführung der Messung der Gerinnungszeit erwünscht ist.

Erfindungsgemäss wird diese Aufgabe hauptsächlich mit einem Kapillarröhrchen gelöst, welches dadurch gekennzeichnet ist, dass

a) es aus einem Kunststoff besteht, und

b) in seinem Innenraum und über seine ganze Länge ein Lyophilisat annähernd gleichmässig verteilt ist, das durch Gefriertrocknung eines Gemisches erzeugt wird, das ein Antikoagulans und wenigstens einen hochmolekularen Zusatz enthält.

Die vom erfindungsgemässen Kapillarröhrchen erzeugte Kapillarkraft bewirkt eine gleichmässige Mischung der ganzen in das Kapillarröhrchen gesaugten Volumens der Blutprobe mit dem im Kapillarröhrchen enthaltenen Antikoagulans. Es wird dadurch sichergestellt, dass kein Teil der Probe koagulieren kann, bevor sie in die Messküvette zur Messung der Gerinnungszeit eingegeben wird.

Das im Kapillarröhrchen enthaltene Lyophilisat bewirkt am Anfang des Saugvorgangs bei der Entnahme der Blutprobe eine Erhöhung der saugenden Kapillarkraft. Das Lyophilisat unterstützt daher die vom Kapillarröhrchen erzeugten Kapillarkraft zur gleichmässigen Mischung der Blutprobe mit dem Antikoagulans und zur vollständigen Füllung des Kapillarröhrchens mit der Blutprobe und des darin aufgelösten Lyophilisates. Durch die vollständige Füllung des Kapillarröhrchens wird das Verhältnis der Probenmenge zur Antikoagulansmenge genau definiert. Durch die beiden soeben erwähnten Wirkungen wird eine höhere Zuverlässigkeit der Messung der Gerinnungszeit ermöglicht.

Das erfindungsgemässe Kapillarröhrchen ermöglicht ausserdem eine erhebliche Vereinfachung bei der Entnahme und Vorbereitung der Blutprobe vor der Messung der Gerinnungszeit auf eine Weise, die die Infektionsgefahr für die ausführende Person erheblich verringert.

Gegenstand der Erfindung ist ferner ein Verfahren zur Herstellung eines erfindungsgemässen Kapillarröhrchens. Dieses Verfahren ist dadurch gekennzeichnet, dass

a) der ganze Innenraum eines aus einem Kunststoff bestehenden Kapillarröhrchens mit einem flüssigen Gemisch gefüllt wird, das ein Antikoagulans und wenigstens einen hochmolekularen Zusatz enthält, und

b) das Gemisch im Innenraum des Kapillarröhrchens gefriergetrocknet wird.

Durch das soeben definierte Herstellungsverfahren wird eine gleichmässige Verteilung des Antikoagulans über die ganze Länge des Kapillarröhrchens erzielt. Dies ermöglicht, allein durch die Wirkung der Kapillarkraft eine gleichmässige Mischung des ganzen in das Kapillarröhrchen gesaugten Probevolumens mit dem darin enthaltenen Antikoagulans zu erzielen.

Im Rahmen der vorliegenden Erfindung ist es ausserdem eine Vorrichtung zur Messung der Gerinnungszeit einer kapillären Blutprobe vorgesehen, welche Vorrichtung eine Küvette zur Photometrischen Messung der Gerinnungszeit einer Blutprobe enthält, welche Küvette aus einem Kunststoff besteht und derer oberen Teil durch eine Trennwand in zwei Räumen aufgeteilt ist, welche Trennwand eine vom Boden der Küvette beabstandete scharfe Kante aufweist. Erfindungsgemäss ist eine solche Vorrichtung dadurch gekennzeichnet,

a) dass sie ein erfindungsgemässes Kapillarröhrchen und einen dazu passenden Deckel enthält, mit dem ein Ende des Kapillarröhrchens verschlossen werden kann, und

b) dass die Küvette in ihrem oberen Teil einen Halter zur Aufnahme des Kapillarröhrchens hat, in welchem Halter das Kapillarröhrchen Parallel zur Langsrichtung der Küvette so angeordnet werden kann, dass ein Teil des Kapillarröhrchens aus der Küvette herausragt.

Die soeben definierte, erfindungsgemässe Vorrichtung bringt alle Vorteile des darin enthaltenen Kapillarröhrchens und ermöglicht insbesondere eine erhebliche Vereinfachung der Entnahme und Vorbereitung der Blutprobe für eine Messung der Gerinnungszeit und dies auf eine Weise, die die Infektionsgefahr für die ausführende Person erheblich verringert.

In einer bevorzugten Ausführungsform der soeben definierten Vorrichtung ist der Halter so ausgebildet, dass durch den Halter das Kapillarröhrchen mit der Küvette kraftschlüssig verbunden werden kann. Dadurch wird eine Erleichterung der Benutzung der Vorrichtung erzielt. In einer bevorzugten Ausführungsform der oben definierten Vorrichtung besteht der Deckel aus einem deformierbaren Material und hat einen Hohlraum, der auch nach Aufsetzen des Deckels auf dem einen Ende des Kapillarröhrchens ein Luftvolumen enthält.

Im folgenden wird anhand der beiliegenden Zeichnungen ein Ausführungsbeispiel der Erfindung beschrieben. Es zeigen

Fig. 1-3 Querschnitte von 3 erfindungsgemässenKapillarröhrchen 11, 12, 13, und zwar je einen Querschnitt entlang der Längsachse des Kapillarröhrchens und einen Querschnitt durch die Linie I-I.

Fig. 4 eine Draufsicht einer Messküvette 41 mit einem darin angeordneten erfindungsgemässen Kapillarröhrchen 81.

Fig. 5 einen Querschnitt der Anordnung gemäss Fig.4 durch die Linie V-V in Fig. 4.

Fig. 6 einen Querschnitt der Anordnung gemäss Fig.4 durch die Linie VI-VI in Fig. 4.

Fig. 7 die Entnahme einer Blutprobe 49 mit dem Kapillarröhrchen 81 der Anordnung gemäss Figuren 4-6.

Fig. 8 einen Querschnitt der Messanordnung gemäss Figuren 4-6, wobei das herausragende Ende des Kapillarröhrchens 81 nach der Probeentnahme gemäss Fig. 7 mit einem Deckel 82 verschlossen ist.

Fig. 9 eine schematische Darstellung der Messküvette und der elektrooptischen Anordnung einer bekannten Vorrichtung zur Messung der Gerinnungszeit.

Fig. 10 einen Querschnitt der Küvette 1 in Fig. 9 und einer damit verbundenen Anordnung 21 zur Erzeugung eines variablen Luftdrucks in der Küvette.

Fig. 11 eine schematische Darstellung von Schritten (1)-(4), die bei der Verwendung der Vorrichtung gemäss Fig. 9 zur Messung der Gerinnungszeit durchgeführt werden.

Figuren 12-14 die Verwendung der Küvette 41 gemäss Fig. 4-6 in einer Messvorrichtung gemäss Figuren 9 und 10.

Fig. 15 einen schematischen Querschnitt einer bevorzugten Ausführungsform der in Fig. 10 dargestellten Anordnung 21 zur Erzeugung eines variablen Luftdruckes.

Fig. 16 eine schematische Darstellung eines typischen Verlaufs des Luftdrucks, der mit der Anordnung gemäss Fig. 15 erzeugbar ist.

Fig. 17 eine Darstellung der Korrelation inzwischen Messergebnissen ,die mit einem konventionellen Mikroquick-Verfahren gewonnen wurden, und Messergebnissen, die mit einem Plasmaquick-Verfahren gewonnen wurden.

Fig. 18 eine Darstellung der Korrelation inzwischen Messergebnissen ,die mit einem anmeldungsgemässen Mikroquick-Verfahren gewonnen wurden, und Messergebnissen, die mit einem Plasmaquick-Verfahren gewonnen wurden.

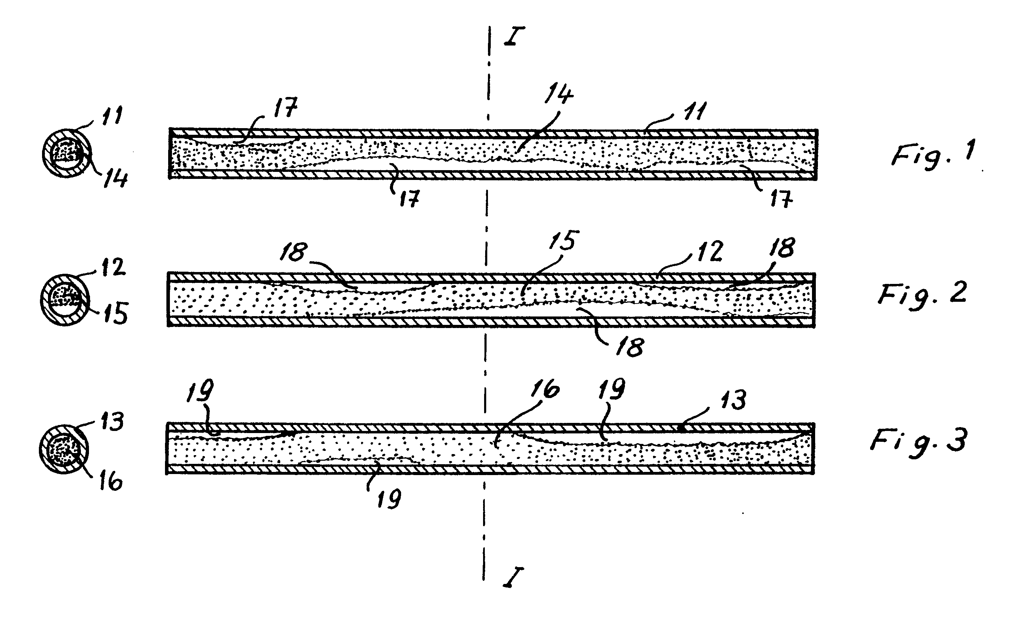

Figuren 1-3 zeigen Querschnitte von drei erfindungsgemässen Kapillarröhrchen 11, 12, 13 zur Entnahme von Blutproben für ein System zur Messung der Gerinnungszeit. Figuren 1-3 zeigen jeweils einen Querschnitt entlang der Längsachse eines der Kapillarröhrchen und einen Querschnitt in einer senkrecht zur Längsachse des Kapillarröhrchens liegenden Ebene (durch die Linie I-I dargestellt).

Leere Kapillarröhrchen 11, 12, 13 sind im Handel erhältlich und werden end-to-end Kapillare genannt. Vorzugsweise werden diese Kapillaren aus Polyethylen (PE) oder aus einem anderen geeigneten Kunststoff gefertigt, z.B. aus Polypropylen (PP), Polyvinylchlorid (PVC) oder Polycarbonate (PC).

Je nach gewünschtem Volumen haben die Kapillarröhrchen 11, 12, 13 folgende Abmessungen:

| Volumen [mikroliter] | Länge [mm] | Aussendurchmesser [mm] | Innendurchmesser [mm] |
|---|---|---|---|
| 25 | 30 | 1.78 - 1.805 | 1.05 - 1.06 |
| 30 | 30 | 1.785 - 1.82 | 1.10 - 1.11 |
| 40 | 30 | 2.10 - 2.14 | 1.25 - 1.30 |
| 50 | 30 | 2.38 - 2.43 | 1.41 - 1.43 |

Ueber die ganze Länge des Innenraums jedes der Kapillarröhrchens 11, 12, 13 ist ein Lyophilisat 14, 15, 16 annähernd gleichmässig verteilt, das durch Lyophilisierung, d.h. durch Gefriertrocknung eines flüssigen Gemisches erzeugt wird, das ein Antikoagulans, d.h. eine gerinnungshemmende Substanz, und wenigstens einen hochmolekularen Zusatz enthält, der als Trägermaterial für das Antikoagulans dient.

Zur Herstellung eines erfindungsgemässen Kapillarröhrchens wird sein ganzer Innenraum mit einem flüssigem Gemisch gefüllt, das ein Antikoagulans und als Trägermaterial wenigstens einen hochmolekularen Zusatzenthält. Das Gemisch wird dann im Innenraum des Kapillarröhrchens gefriergetrocknet. Durch die Gefriertrocknung wird innerhalb jedes der Kapillarröhrchen ein Lyophilisat 14, 15, 16 gebildet. Dabei entstehen in den Kapillarröhrchen leere Räume 17, 18, 19.

Das Verfahren zur Herstellung des Lyophilisates in den Kapillarröhrchen umfasst folgende Schritte:

1. Die Lösung wird vorbereitet, mit der die Kapillarröhrchen gefüllt werden. Diese Lösung weist z.B. folgende Zusammensetzung auf:

| - 0.011 mol/l | Trinatriumcitrat |
|---|---|
| - 3.000 g/l | Gelatine |

2. Eine Vielzahl Kapillarröhrchen werden mittels eintauchen in die Lösung vollständig mit Lösung gefüllt.

3. Die mit Lösung gefüllten Kapillarröhrchen werden in waagrechter Lage auf eine vorgekühlte Platte eines Lyophilisators gelegt und sie bleiben dort mindestens 15 Minuten. Dadurch wird ein schnelles Durchfrieren der Lösung in den Kapillarröhrchen erzielt. Die Temperatur der vorgekühlten Platte muss niedriger als - 35° C sein.

4. Die Gefriertrocknung erfolgt in einem üblichen Lyophilisator, der vorzugsweise über eine Steuerung gesteuert werden werden kann. Im Lyophilisator erfolgt die Lyophilisierung in 2 Stufen. Zuerst wird eine Primärtrocknung (Sublimation) und anschliessend eine Sekundärtrocknung durchgeführt. Die Primärtrocknung wird bei einem Druck von 400 Mikrobar während 12 Stunden durohgeführt, wobei die Temperatur des Produktes über mindestens 6 Stunden niedriger als - 20° C ist. Die Sekundärtrocknung wird bei einem Druck von 50 Mikrobar während mindestens 4 Stunden durchgeführt, wobei die Temperatur des Produktes + 35° C beträgt.

Als Antikoagulans wird vorzugsweise Trinatriumcitrat in einer Konzentration von 0,011 mol/l verwendet. Als Antikoagulans können z.B. auch Aethylendiamin-Tetraessigsäuure-Dinatriumsalz (EDTA) oder Natriumfluorid verwendet werden. Die als Antikoagulans verwendete Substanz wird in einer wässerigen Lösung eingesetzt, wobei die Konzentration 1/10 der für venöse Blutentnahme entsprechenden Konzentration entspricht.

Als Trägermaterial für das Antikoagulans werden vorzugsweise hochmolekulare Zusätze wie z.B. Fibrinogen vom Rind oder wasserlösliche Gelatine verwendet. Als Trägermaterial können auch z.B. menschliches Fibrinogen, Albumin vom Rind, Polyethylenglykol PEG 6000 oder Polyvinylpyrolidone 20 bzw. 30 verwendet werden. Die Trägermaterialien werden als wässerige Lösungen vorzugsweise im Konzentrationsbereich von 0.1 bis 3.0 % eingesetzt.

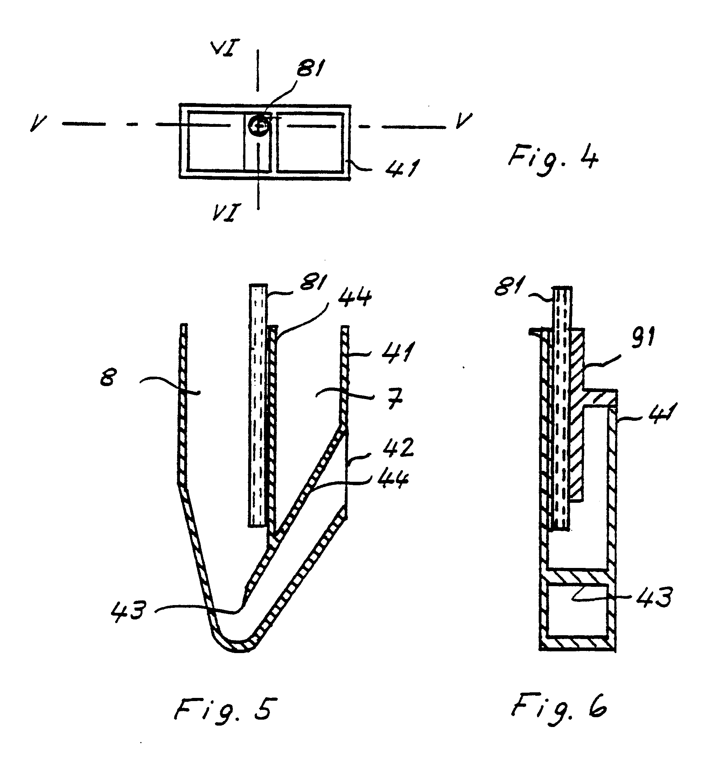

Ein erfindungsgemässes Kapillarröhrchen gemäss einer der Figuren 1-3 wird vorzugsweise in Kombination mit einer dazu passenden Messküvette in einer Vorrichtung zur Messung der Gerinnungszeit einer Blutprobe verwendet. Eine hierfür geeignete Messküvette wird nachstehend anhand der Fig. 4-6 beschrieben.

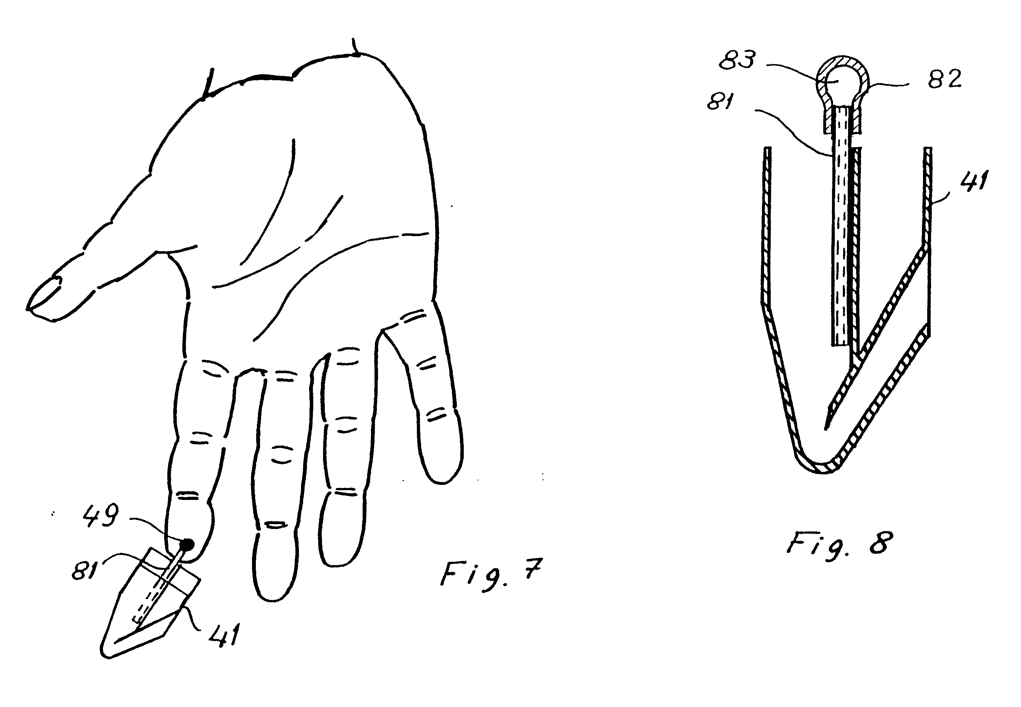

Fig. 4 zeigt eine Draufsicht einer Messküvette 41 mit einem darin angeordneten erfindungsgemässen Kapillarröhrchen 81. Fig. 5 zeigt einen Querschnitt der Anordnung gemäss Fig.4 durch die Linie V-V in Fig. 4. Fig. 6 zeigt einen Querschnitt der Anordnung gemäss Fig.4 durch die Linie VI-VI in Fig. 4.

Die Messküvette 41 besteht aus einem Material, das die Durchführung von optischen, vorzugsweise Photometrischen Messungen ermöglicht, z.B. aus einem geeigneten Kunststoff. Die Küvette 41 muss nicht zwingend die in Figuren 4-6 gezeigten Form haben. Wesentlich ist hingegen, dass sie eine Trennwand 44 mit einer vom Boden beabstandeten scharfen Kante 43 hat. Diese Trennwand trennt den oberen Teil der Küvette in zwei Räumen 7 und 8. Wie aus Fig. 6 ersichtlich, hat die Küvette 41 in ihrem oberen Teil einen Halter 91 zur Aufnahme eines erfindungsgemässen Kapillarröhrchens 81. Das Kapillarröhrchen 81 wird im Halter 91 vorzugsweise parallel zur Längsrichtung der Küvette 41 so angeordnet, dass ein Teil des Kapillarröhrchens aus der Küvette herausragt. Der Halter 91 ist vorzugsweise so ausgebildet, dass das Kapillarröhrchen 81 durch ihn mit der Küvette 41 kraftschlüssig verbunden werden kann.

Die Fig. 7 zeigt die Entnahme einer Blutprobe 49 mit einem erfindungsgemässen Kapillarrörchen 81, der wie soeben beschrieben in einer Messküvette 41 angeordnet ist. Nach der Punktierung einer Fingerkuppe eines Patienten mit einer Lanzette werden die austretenden Blutstropfen für die Untersuchung verwendet. Die vorgeschriebene Menge Blut (10 bis 50 mikroliter) wird mit einem erfindungsgemässen Kapillarröhrchen aufgezogen, in dem die Spitze des horizontal gehaltenen Röhrchens in die austretenden Blutstropfen gehalten wird. Bei dieser Entnahme der Blutprobe wird das Blut ausschliesslich durch die vom erfindungsgemässen Kapillarröhrchen erzeugte Kapillarkraft in den Innenraum des Kapillarröhrchens aufgezogen.

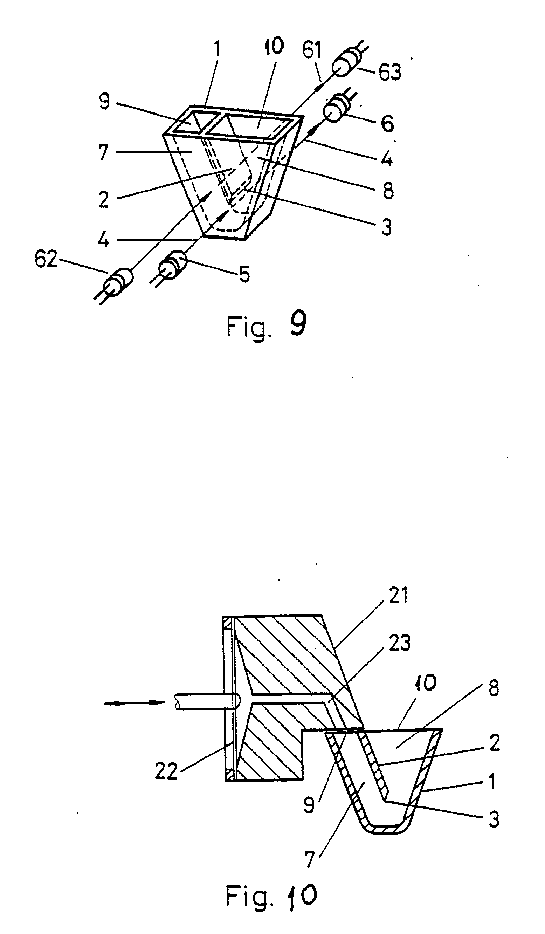

Wie in der Fig. 8 dargestellte wird unmittelbar nach der Entnahme der Blutprobe das aus der Messküvette 41 herausragende Ende des Kapillarröhrchens 81 mit einem Deckel 82 verschlossen. Der Deckel besteht vorzugsweise aus einem weichen deformierbaren Material, z.B. aus Brombutylkautschuk, und hat einen Hohlraum 83, der auch nach Aufsetzen des Deckels auf dem einen Ende des Kapillarröhrchens ein Luftvolumen enthält.

Die Uebertragung der in dem Kapillarröhrchen 81 enthaltenen Probe in die Messküvette 41 wird durch den Druckstoss ausgelöst, der beim Aufsetzen des Deckels auf dem einen Ende des Kapillarröhrchens erzeugt wird. Diese Uebertragung der Probe erfolgt vorwiegend unter Einwirkung der Schwerkraft, wenn dass Kapillarröhrchen von der waagrechten Lage bei der Probeentnahme in einer vertikalen Lage gebracht wird. Dabei fliesst die Probe aus dem Kapillarröhrchen 81 und wird im unteren Teil der Messküvette 41 aufgenommen. Zur vollständigen Abgabe der in dem Kapillarröhrchen enthaltene Probe in die Messküvette 41 wird der obere, den Hohlraum 83 enthaltende Teil des Deckels 82 mit den Fingern zusammengedrückt. Durch den dadurch erzeugten Druckstoss wird sichergestellt, dass die ganze Probe in die Messküvette übertragen wird. Dies ist für die Reproduzierbarkeit der Messungen wichtig.

Nach der soeben erwahnten Uebertragung der Probe in der Messküvette kann das leere Kapillarröhrchen - mit oder ohne Deckel - in der Messküvette belassen oder aus dieser entfernt werden. Die Messküvette wird dann in einer Vorrichtung zur Messung der Gerinnungszeit eingesetzt. Der Aufbau und die Funktionsweise dieser Vorrichtung werden nachstehend anhand der Figuren 9 bis 18 beschrieben. In diesen Figuren wird das erfindungsgemässe Kapillarröhrchen nicht gezeigt, weil es für die eigentliche Durchführung der photometrischen Messung der Gerinnungszeit nicht notwendig ist.

Fig. 9 zeigt eine in der Messvorrichtung eingesetzte Messküvette 1 und eine schematische Darstellung der elektrooptische Anordnung der Messvorrichtung. Die Küvette 1 hat zwar nicht die gleiche Form wie die Küvette 41 gemäss Figuren 4-6, hat aber im wesentlichen eine sehr ähnliche Struktur. Wie die Küvette 41 gemäss Figuren 4-6 hat auch die Küvette 1 hat eine Trennwand 2 , die den oberen Teil der Küvette in zwei Räumen 7 und 8 trennt und die eine vom Boden der Küvette beabstandete scharfe Kante 3 aufweist.

Die in Fig. 9 dargestellte elektrooptische Anordnung enthält eine Lichtquelle 5, z.B. eine lichtaussende-de Diode, und einen Lichtempfänger 6, z.B. eine Photodiode, in der gezeigten Anordnung. Mit diesen Komponenten wird ein Lichtstrahl 4 erzeugt bzw. empfangen. der die Küvette durchquert, wobei er nahe und unterhalb der Kante 3 verläuft um die Messung der Extinktion des vom Lichtstrahl 4 erfassten Inhalts der Küvette zu ermöglichen. Der Verlauf des Lichtstrahls 4 wird so gewählt, dass ein sich an der Kante 3 bildendes Gerinnsel 33 vom Lichtstrahl 4 erfasst wird.

Die elektrooptische Anordnung enthält ausserdem eine Lichtquelle 62, z.B. eine lichtaussendende Diode 62, und einen Lichtempfänger 63, z.B. eine Photodiode 63 in der gezeigten Anordnung. Mit diesen Komponenten wird ein Lichtstrahl 61 erzeugt bzw. empfangen, der die Küvette durchquert, wobei er oberhalb der Kante 3 verläuft, um eine Messung der Extinktion des vom Lichtstrahl 61 erfassten Inhalts der Küvette zu ermöglichen. Der Verlauf des Lichtstrahls 61 wird so gewählte, dass die maximal vorgesehene Probenmenge, die in die Küvette eingeführt wird, vom Lichtstrahl 61 nicht erfasst wird, dass aber nach Zugabe eines für die Gerinnungsmessung erforderlichen Reagenz auch beim minimal vorgesehenen Volumen des Probe-Reagenz-Gemisches, dieses vom Lichtstrahl 61 erfasst wird.

Um den Einfluss des Umgebungslichtes auf die Extinktionsmessungen mit den Lichtstrahlen 4 und 61 zu verringern, werden als Lichtempfänger 6 bzw. 63 vorzugsweise Photodioden mit eingebautem Infrarotfilter verwendet, deren Durchlassbereich von ca. 900 bis ca. 1000 nm geht und eine minimale Dämpfung bei ca. 940 nm hat. Dadurch wird z.B. die Empfindlichkeit der Messungen auf eine Umgebungsbeleuchtung mit Glühlampenlicht etwa 5 mal kleiner.

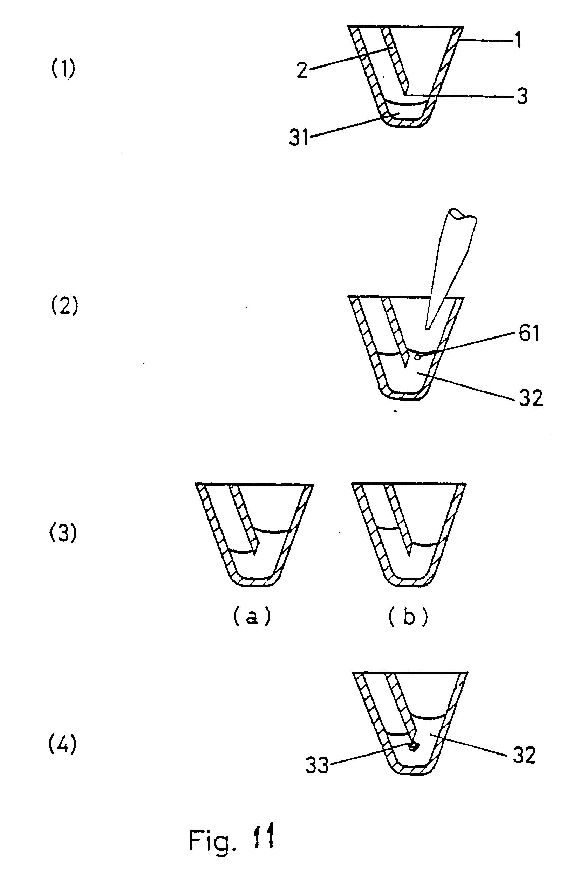

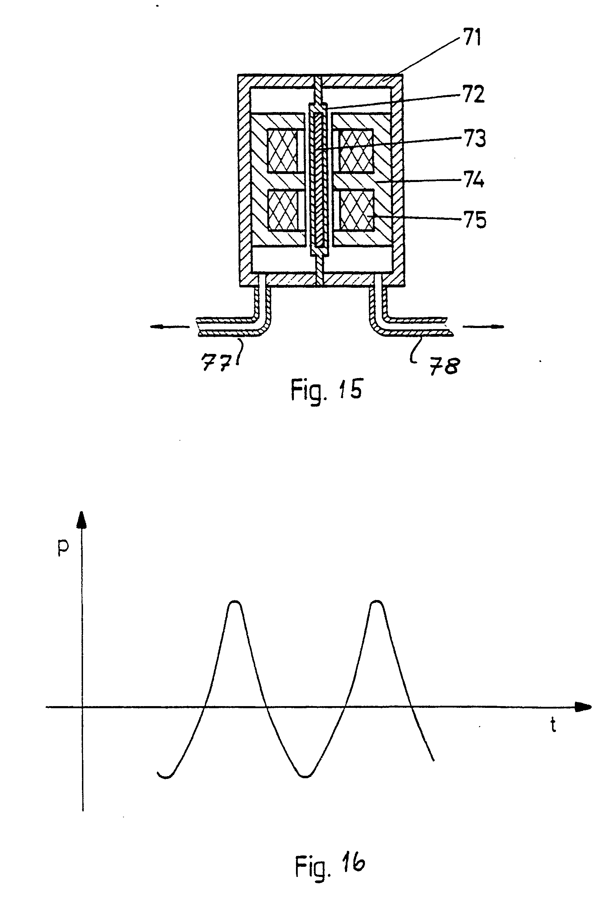

Wie in Fig. 10 gezeigt ist der Raum 7 im oberen Teil der Küvette 1 durch eine Oeffnung 9 mit einer Anordnung 21 verbunden, mit der ein variabler, auf den Inhalt der Küvette wirkenden Luftdruck erzeugt werden kann. Durch diesen Druck wird in der erfindungsgemässen Vorrichtung das Probe-Reagenz-Gemisch auf die Weise bewegt, die nachstehend anhand der Fig. 11 (3) erläutert ist. Wie nachstehend anhand der Fig. 15 und 16 beschrieben, kann die Anordnung 21 z.B. eine vibrierende Membrane 22 als Pumpelemente enthalten. Der damit erzeugte Luftdruck gelangt durch ein Kanal 23 und durch die Oeffnung 9 in die Küvette 1. Die Küvette hat eine Oeffnung 10, die im Betrieb offen bleibt.

Wie in Fig. 11 gezeigt, werden bei der Verwendung der erfindungsgemässen Küvette zur Messung der Gerinnungszeit einer Blutprobe folgende Schritte durchgeführt:

(1) Das vorgesehene Ansatzvolumen der aufbereiteten Blut- oder Plasmaprobe 31, der von einem Kapillarröhrchen wie 81 in Fig. 8 abgegeben und im unteren Teil der Küvette 1 aufgenommen wird, wird dort inkubiert. Wie in der Fig. 11 gezeigte wird die Probenmenge vorzugsweise so klein gewählt, dass sie die Kante 3 nicht erreicht, so dass Luft zwischen der Oberfläche der Probe und der Kante 3 frei strömen kann. Der von der Anordnung 21 erzeugte Luftdruck kann also keine Bewegung der Probe bewirken, und die Probe steht still, solange die vorgesehene Reagenzmenge noch nicht in die Küvette eingeführt wird.

(2) Das vorgesehene Ansatzvolumen des Reagenz wird in die Küvette eingeführt. Die Probe- bzw. Reagenzmenge werden so gewählt, dass die Oberfläche des so gebildeten Probe-Reagenz-Gemisches auf jeden Fall oberhalb der Kante 3 und des Lichtstrahls 61 liegt. Dadurch werden folgende Effekte erzielt, sobald das Reagenz in die Küvette eingeführt wird:

(3) Das Probe-Reagenz-Gemisch 32 wird vom Lichtstrahl 61 erfasst. Dieser Zeitpunkt markiert den Beginn der Messung der Gerinnungszeit. Dieser Zeitpunkt wird mit an sich bekannten Mittel der elektronischen Signalverarbeitung registriert. Durch die Wirkung des oben erwähnten, mit den in Fig. 10 gezeigten Mitteln erzeugten Luftdrucks fliesst nun das Probe-Reagenz-Gemisch und bewegt sich dabei zwischen den in Fig. 11(3) gezeigten Positionen (a) und (b) hin und her. Dadurch wird zugleich die Probe mit dem Reagenz gut gemischt und ein Fliessen des Gemisches um die scharfe Kante 3 der Küvette bewirkt.

(4) Durch die in (3) gezeigte relative Bewegung der Flüssigkeit in bezug auf die Kante 3 bildet sich im Augenblick der Gerinnung ein Gerinnsel 33 an der Kante 3. Da dieses Gerinnsel vom Lichtstrahl 4 erfasst wird, ergibt sich eine Plötzliche und deutliche Aenderung der mit diesem Lichtstrahl gemessenen Extinktion. Durch die Feststellung dieser Aenderung wird der Zeitpunkt der Gerinnung und somit die Gerinnungszeit eindeutig und genau gemessen. Für diese Feststellung und für die Anzeige der ermittelten Werte werden an sich bekannte Mittel der elektronischen Signalverarbeitung bzw. Datenanzeige verwendet.

In Abweichung von dem oben beschriebenen Verfahren, und sofern dadurch die Messung nicht beeinträchtigt wird, kann die Probenmenge so gross gewählt werden, dass auch wenn die Probe sich allein in der Küvette befindet, ihre Oberfläche oberhalb der Kante 3 liegt. In diesem Fall wird vor der Zugabe vom Reagenz die Probe vom Lichtstrahl 4 erfasst und durch die Wirkung des oben erwähnten Luftdruckes bewegt, analog wie in Fig. 11(3) für das Probe-Reagenz-Gemisch gezeigt.

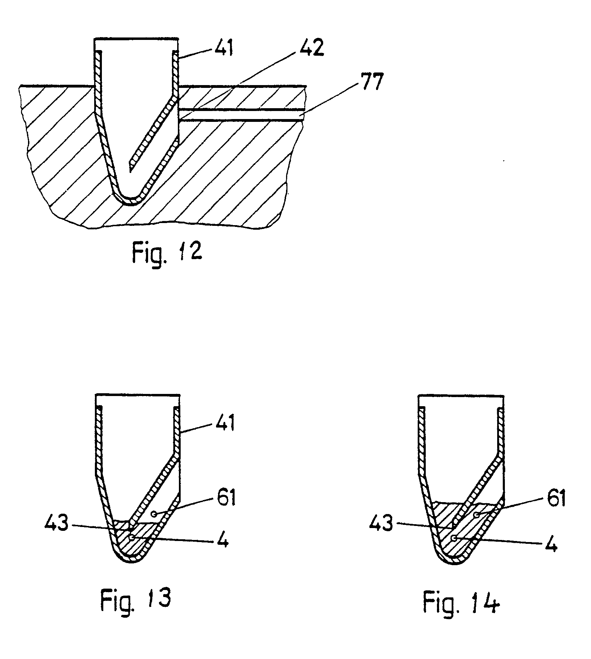

Die Figuren 12 bis 14 zeigen die Verwendung der Küvette 41 gemäss Fig. 4-6 in einer Messvorrichtung gemäss Fig. 9-10.

Wie in Fig. 12 gezeigte wird der Küvette 41 der variable Luftdruck durch eine seitliche Oeffnung 42 zugeführte, die über eine Leitung 77 mit der Luftdruck erzeugenden Anordnung verbunden ist.

Fig. 13 zeigt die Küvette 41 wenn sie nur die Probe enthält. Fig. 14 zeigt die Küvette 41 wenn sie das Probe-Reagenz-Gemisch enthält. In Fig. 13 und 14 ist die Lage der Lichtstrahlen 4 bzw. 61 mit je einem kleinen Kreis markiert.

Die in den Figuren 13 und 14 dargestellte Verwendung der Küvette 41 ist gleich wie oben anhand der Fig. 11 beschrieben.

Fig. 15 zeigt schematisch einen Querschnitt einer bevorzugten Ausführungsform der Anordnung 21 (in Fig. 10) zur Erzeugung des variablen Luftdrucks. Diese Anordnung ist in einem Gehäuse 71 enthalten. Darin ist eine Membrane 72 angeordnet, die einen Eisenblechkern 73 hat. Mit einer einen Magnetkern 74 und eine Magnetspule 75 enthaltenden elektromagnetischen Antriebseinrichtung, der ein geeigneter Wechselstrom zugeführt wird, wird eine Schwingung der Membrane 72 bewirkt. Dadurch entsteht im Gehäuse 71 ein variabler Luftdruck der zwei Küvetten der erfindungsgemässen Vorrichtung über Leitungen 77 bzw. 78 zugeführt wird.

Fig. 16 zeigt schematisch einen typischen zeitlichen Verlauf des Luftdrucks, der mit der in Fig. 15 gezeigten Anordnung erzeugbar ist. Der Luftdruck variiert zwischen +2 und -2 Millibar, und sein Verlauf hat annähernd die Form einer Sinusschwingung mit einer Frequenz von ca. 40 Hz.

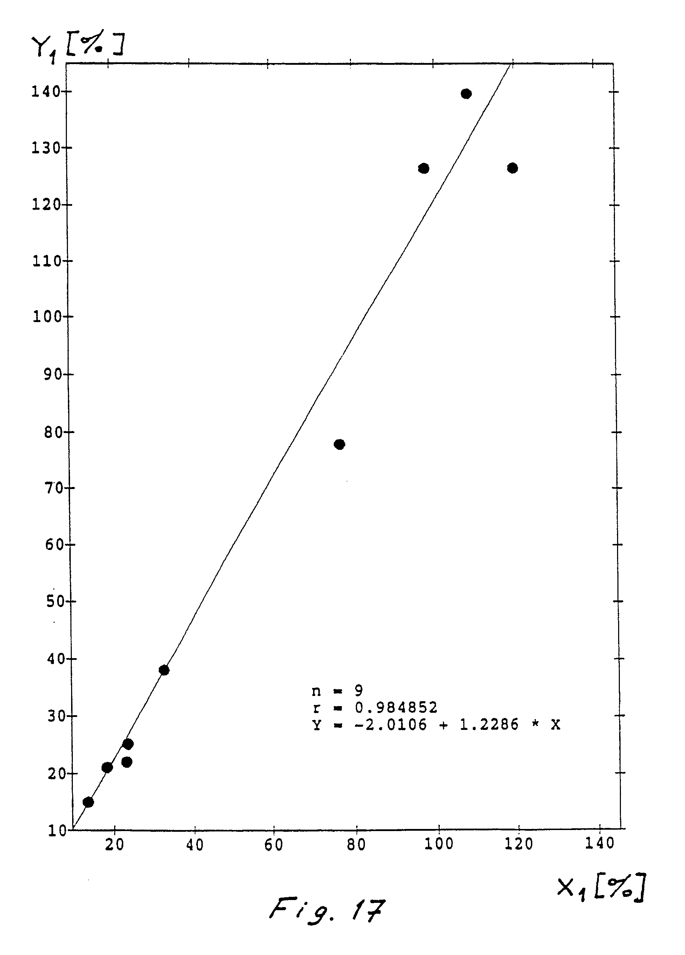

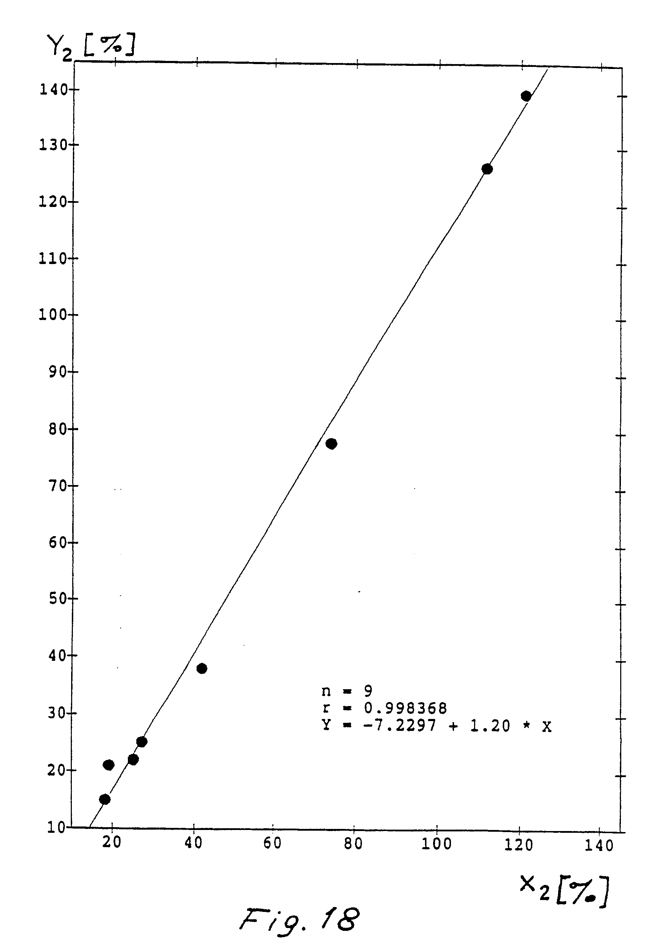

Anhand der in den Figuren 17 und 18 dargestellten Messergebnisse wird nachstehend gezeigt, dass wenn bei einem sogenannten Mikroquick-Verfahren zur Bestimmung der Gerinnungszeit die Entnahme und Aufbereitung der Blutprobe mit einem erfindungsgemässen Kapillarröhrchen durchgeführt wird, die erzielte Korrelation mit entsprechenden Messergebnissen eines sogenannten Plasmaquick-Verfahren, welche Messergebnisse als genau und zuverlässig anerkannt sind, besser ist, als wenn die Entnahme und Aufbereitung der Blutprobe mit dem bisher üblichen Verfahren durchgeführt wird, der in der Einleitung dieser Beschreibung beschrieben wurde.

Es sei an dieser Stelle darauf hingewiesen, dass die Verfahren zur Entnahme und Aufbereitung der Probe bei den oben erwähnten Verfahren zur Bestimmung der Gerinnungszeit erheblich voneinander abweichen. Dies geht aus der nachstenden Beschreibung dieser Verfahren hervor.

Plasmaquick-Verfahren

Das Plasmaquick-Verfahren ist ein Verfahren zur Bestimmung der Thromboplastinzeit mit venösem Citratplasma. Beim konventionellen Plasmaquick-Verfahren wird Plasma nach folgender Vorschrift gewonnen:

Mit Hilfe einer sterilen 2-ml-Einwegspritze aus Kunststoff, die genau 0,2ml-sterile Natriumcitrat-Lösung 0,1 mol/l enthält, entnimmt man durch Venenpunktion Blut bis 2,0-ml-Marke, zieht den Stempel zurück und mischt den Inhalt rasch und vollständig durch mehrmaliges Neigen der Spritze.Dieses Citratblut wird während 15 Minuten bei 3000 U/min (ca. 1600 x g) zentrifugiert.

Die Gerinnungsbestimmungen, sind möglichst anschliessend, auf jeden Fall aber innerhalb von 2 Stunden nach der Blutentnahme durchzuführen. Das so gewonnene Plasma wird in Einwegröhrchen aus Kunststoff bei 15-25° C aufbewahrt.

Die Messung der Gerinnungszeit wird z.B. mit dem Messgerät <COBAS> Fibro durchgeführt, dessen Prinzipiellen Aufbau und Funktionsweise oben anhand der Figuren 9 - 16 beschrieben ist. Zur Durchführung der Messung mit <COBAS> Fibro wird ausserdem eine <COBAS> Fibro-Messküvette, automatische Mikropipetten für Volumina von 100 und 200 Mikroliter und eine Stoppuhr benötigt. Vor der Durchführung der Messung wird ein Volumen von 0,1 ml des nach der obigen Vorschrift gewonnenen Plasma unter Verwendung einer vorgewärmten Küvette und des Thermoblocks des <COBAS> Fibro während 2 Minuten bei 37° vorgewärmt. Dann werden 0,2 ml Reagenzlösung zugegeben. Danach wird die Messung in der oben anhand der Figuren 9 - 16 beschriebene Weise durchgeführt.

Konventionelles Mikroquick-Verfahren

Das konventionelle Mikroquick-Messverfahren ist ein Verfahren zur Bestimmung der Thromboplastinzeit mit kapillärem Citratblut. Beim konventionellen Mikroquick-Verfahren wird die Entnahme der Blutprobe und ihre Vorbereitung vor der Gerinnungszeitmessung nach folgender Vorschrift durchgeführt:

1. Citratlösung in einer Kapillare bis zur 0.005 ml-Marke aufziehen.
2. Finger (vorzugsweise Mittel oder Ringfinger der linken Hand) mit Alkohol reinigen und mit einer Lanzette einen Stich setzen. Den ersten Blutstropfen abwischen.
3. In die mit Citratlösung versehene Kapillare Blut bis zur 0.050 ml-Marke einfliessen lassen. Sofort in ein Glas-oder Plastikröhrchen - oder direkt in die Messküvette eines Gerinnungszeitmessgeräts - gegen dessen Boden ausblasen (Schaumbildung vermeiden) und durch kreisende Bewegung mischen.
4. Die Gerinnungsmessungen sind, wenn immer möglich, innerhalb 10 Minuten, jedoch spätestens 60 Minuten nach der Blutentnahme durchzuführen, wobei das Citratblut unter keinen Umständen in der Kapillare liegen bleiben darf.

Die Messung der Gerinnungszeit wird z.B. mit dem Messgerät <COBAS> Fibro durchgeführt, dessen prinzipiellen Aufbau und Funktionsweise oben anhand der Figuren 9 - 16 beschrieben ist. Zur Durchführung der Messung mit <COBAS> Fibro wird ausserdem eine <COBAS> Fibro-Messküvette, eine automatische Mikropipette für ein Volumen von 250 Mikroliter und eine Stoppuhr benötigt. Vor der Durchführung der Messung wird ein Volumen von 0,050 ml des nach der obigen Vorschrift gewonnenen Kapillarcitratbluts unter Verwendung des Thermoblocks des <COBAS> Fibro während 2 Minuten bei 37° vorgewärmt. Dann werden 0.250 ml Reagenzlösung zugegeben. Danach wird die Messung in der oben anhand der Figuren 9 -16 beschriebene Weise durchgeführt.

Vergleich der Messergebnisse mit dem konventionellen Mikroquick- bzw. mit dem Plasmaquick-Verfahren

In der Fig. 17 sind Messergebnisse durch Punkte dargestellt, die mit einer Gruppe von Proben einerseits mit einem konventionellen Mikroquick-Verfahren, andererseits mit einem Plamaquick-Verfahren gewonnen wurden. Die $X_1$-Messwerte in % wurden mit dem konventionellen Mikroquick-Verfahren gewonnen. Die $Y_1$-Messwerte in % wurden mit dem Plasmaquick-Verfahren gewonnen.

Zur Erzielung vergleichbarer Messergebnisse wurde sowohl beim konventionellen Mikroquickverfahren, wie auch beim Plasmaquickverfahren das gleiche Reagenz, z.B. CRB-Thromboplastin <ROCHE>, verwendet. Die Messung der Gerinnungszeit wurde für beide Verfahren mit dem Gerinnungszeit-Messgerät <COBAS> Fibro <ROCHE> durchgeführt.

In der Fig.17 sind die Anzahl n der untersuchten Proben, die Regressionsgleichung der Form $y = a + bx$ und der Korrelationskoeffizient r angegeben.

Erfindungsgemässes Mikroquick-Verfahren

Bei diesem Verfahren wird die Entnahme und Aufbereitung der Blutpprobe wie oben anhand der Figuren 7 und 8 beschrieben. Die Messung der Gerinnungszeit wird z.B. mit dem Messgerät <COBAS> Fibro in gleicher Weise durchgeführt, wie oben für das konventionelle Mikroquick-Verfahren beschrieben.

Vergleich der Messergebnisse mit dem erfindungsgemässen Mikroquick- bzw. mit dem Plasmaquick-Verfahren

In der Fig. 18 sind Messergebnisse durch Punkte dargestellt, die mit einer Gruppe von Proben einerseits mit einem erfindungsgemässen Mikroquick-Verfahren, andererseits mit einem Plasmaquick-Verfahren gewonnen wurden. Die $X_2$-Messwerte in % wurden mit dem erfindungsgemässen Mikroquick-Verfahren gewonnen. Die $Y_2$-Messwerte in % wurden mit dem Plasmaquick-Verfahren gewonnen. Von den in der Fig. 18 dargestellten Messpunkte haben 2 identische $X_2$- und $Y_2$-Werte. Deshalb sind in der Fig. 18 für 9 untersuchte Proben nur 8 Messpunkte ersichtlich.

Zur Erzielung vergleichbarer Messergebnisse wurde sowohl beim erfindungsgemässen Mikroquickverfahren, wie auch beim Plasmaquickverfahren das gleiche Reagenz, z.B. CRB-Thromboplastin <ROCHE>, verwendet. Die Messung der Gerinnungszeit wurde für beide Verfahren mit dem Gerinnungszeit-Messgerät <COBAS> Fibro <ROCHE> durchgeführt.

In der Fig.18 sind die Anzahl n der untersuchten Proben, die Regressionsgleichung der Form y = a + bx und der Korrelationskoeffizient r angegeben.

Es ist aus einem Vergleich der Figuren 17 und 18 ersichtlich, dass mit dem erfindungsgemässen Kapillarröhrchen bzw. mit dem erfindungsgemässen Mikroquick-Verfahren eine bessere Korrelation in bezug auf die mit dem Plasmaquick-Verfahren erzielten Messwerte erreicht wird, und dass dies auch im Bereich der relativ hohen Messwerte der Fall ist.


## Ansprüche

1. Kapillarröhrchen (11, 12, 13) zur Entnahme von kapillären Blutproben für ein System zur Messung der Gerinnungszeit,
dadurch gekennzeichnet, dass
a) es aus einem Kunststoff besteht, und
b) in seinem Innenraum und über seine ganze Länge ein Lyophilisat (14, 15, 16) annähernd gleichmässig verteilt ist, das durch Gefriertrocknung eines Gemisches erzeugt wird, das ein Antikoagulans und wenigstens einen hochmolekularen Zusatz enthält.

2. Verfahren zur Herstellung eines Kapillarröhrchens gemäss Anspruch 1, dadurch gekennzeichnet, dass
a) der ganze Innenraum eines aus einem Kunststoff bestehenden Kapillarröhrchens mit einem flüssigen Gemisch gefüllt wird, das ein Antikoagulans und wenigstens einen hochmolekularen Zusatz enthält. und
b) das Gemisch im Innenraum des Kapillarröhrchens gefriergetrocknet wird.

3. Vorrichtung zur Messung der Gerinnungszeit einer kapillären Blutprobe, welche Vorrichtung eine Küvette (41) zur photometrischen Messung der Gerinnungszeit einer Blutprobe enthält, welche Küvette aus einem Kunststoff besteht und derer oberen Teil durch eine Trennwand (44) in zwei Räumen (7, 8) aufgeteilt ist, welche Trennwand eine vom Boden der Küvette beabstandete scharfe Kante (43) aufweist, dadurch gekennzeichnet,
a) dass sie ein erfindungsgemässes Kapillarröhrchen (11, 12, 13, 81) und einen dazu passenden Deckel (82) enthält, mit dem ein Ende des Kapillarröhrchens verschlossen werden kann, und
b) dass die Küvette (41) in ihrem oberen Teil einen Halter (91) zur Aufnahme des Kapillarröhrchens hat, in welchem Halter das Kapillarröhrchen parallel zur Längsrichtung der Küvette so angeordnet werden kann, dass ein Teil des Kapillarröhrchens aus der Küvette herausragt.

4. Vorrichtung gemäss Anspruch 3, dadurch gekennzeichnet, dass der Halter (91) so ausgebildet ist, dass das Kapillarröhrchen (81) durch ihn mit der Küvette (41) kraftschlüssig verbunden werden kann.

5. Vorrichtung gemäss Anspruch 3, dadurch gekennzeichnet, dass der Deckel (82) aus einem deformierbaren Material besteht und einen Hohlraum (83) hat, der auch nach Aufsetzen des Deckels auf dem einen Ende des Kapillarröhrchens ein Luftvolumen enthält.

Fig. 1

Fig. 2

Fig. 3

EP 0 421 175 A1

Fig. 4

Fig. 5

Fig. 6

Fig. 7

Fig. 8

EP 0 421 175 A1

Fig. 9

Fig. 10

(1)

2

1

3

31

(2)

61

32

(3)

(a)          (b)

(4)

33          32

Fig. 11

Fig. 12

Fig. 13

Fig. 14

Fig. 15

Fig. 16

Fig. 17

Fig. 18

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | US-A-3 951 606 (R.H. MOYER)<br>* Patentansprüche 1,9,14,15 *<br>- - - | 1,3 | G<br>01 N 21/03<br>G 01 N 33/48<br>A 61 B 5/14 |
| A | FR-A-2 076 366 (G. CHEMLA et al.)<br>* Patentansprüche 1-3 *<br>- - - | 1 | |
| A | GB-A-2 100 427 (F. HOFFMANN-LA ROCHE)<br>* Patentansprüche 1-9 *<br>- - - | 3 | |
| A | EP-A-0 254 246 (PERSONAL DIAGNOSTICS)<br>* Zusammenfassung *<br>- - - - - | 3 | |

| RECHERCHIERTE SACHGEBIETE (Int. Cl.5) |
|---|
| G 01 N<br>A<br>61 B<br>B 01 L |

Der vorliegende Recherchenbericht wurde für alle Patentansprüche erstellt

| Recherchenort | Abschlußdatum der Recherche | Prüfer |
|---|---|---|
| Den Haag | 10 Januar 91 | VAN DEN BULCKE E. |